# EUROPEAN PATENT APPLICATION

(11) **EP 1 223 387 A2**
(43) Date of publication of application: **17.07.2002**
(21) Application number: 02000666.4
(22) Date of filing: 11.01.2002
(51) Int. Cl.: F24F 3/16

(54) **Air treatment system**

(30) Priority: 11.01.2001 GB 0100714
(71) Applicant: Ozone Industries Limited, Farnborough, Hants GU14 0NR (GB)
(72) Inventor: Martensson, Nils, Chobham, GU24 8JE (GB)
(74) Representative: Harman, Michael Godfrey

(57) **Abstract**

An air treatment system includes an ozone generator and a volatile oil vaporiser, air being taken up and mixed with the ozone and/or volatile oil. The ozone and volatile oil may be provided in separate or separable units, the outlets of the units directing the ozone and volatile oil together. Alternatively, the ozone from one unit may be taken up and mixed with the volatile oil produced by the other unit. The production of volatile air and the production of ozone may be independently controlled.

## Description

The present invention relates to an air treatment system, in particular for air in buildings occupied by people.

A known air freshening unit holds a quantity of volatile scent, and also includes a heating means, which obtains its power from a standard three pin plug, the pins extending directly from the unit. When the unit is plugged into a standard 13 amp three hole socket (i.e. the UK 'mains' supply) and the socket is switched on, the heating means causes the scent to evaporate. The scent vapour diffuses into the air of the room in which the plug is placed and imparts a pleasant smell to the room and masks unpleasant odours already present.

The object of the present invention is to provide an improved and flexible system for treating a volume of air

According to the present invention there is provided an air treatment system including
an ozone generator contained within a first housing,
a volatile oil vaporiser contained within a second housing,
each housing including an inlet and an outlet,
the two housings being separate or separable from each other
such that in operation air enters through at least one of the inlets, and ozone and/or volatile oil exits with the air through at least one of the outlets.

According to another aspect of the present invention there is provided an air treatment system including
an ozone generator,
a volatile oil vaporiser,
housing means for shielding and/or supporting the ozone generator and the volatile oil vaporiser, the housing means including one or more inlets and one or more outlets, and
such that in operation air enters through the inlet, and ozone and/or volatile oil exits with the air through the outlet.

Preferably the production of air containing volatile oil and the production of ozone may be independently controlled.

Preferably the ozone generator is contained within a first housing, and the volatile oil vaporiser is contained within a second housing, the two housings being separate or separable from each other and each housing including plug means. The air containing ozone exits from a first outlet, and air containing volatile oil exits from a second outlet, the outlets directing their air flows together so as to mix in the surrounding air.

According to a further aspect of the present invention there is provided an ozone generator adapted to co-operate with a volatile oil vaporiser as defined above.

According to a further aspect of the present invention there is provided a volatile oil vaporiser adapted to co-operate with an ozone generator as defined in any previous claim.

According to a yet further aspect of the present invention there is provided a method of treating air in a room or the like by generating a first air stream containing ozone, generating a second air stream containing volatile oil, and directing the air streams so as to converge and mix.

Preferably, the volatile oil includes terpenes.

It will be seen that such a system conveniently increases the effectiveness of treating the air in a room and affords a great deal of flexibility as to how the air is to be treated.

The invention will now be described, by way of example, with reference to the drawings, of which
Figure 1 shows a perspective view of the system,
Figure 2 shows a different perspective view of a sub-unit of the system,
Figure 3 shows a section of a sub-unit of the system, and
Figure 4 shows a section of another embodiment of the sub-unit.

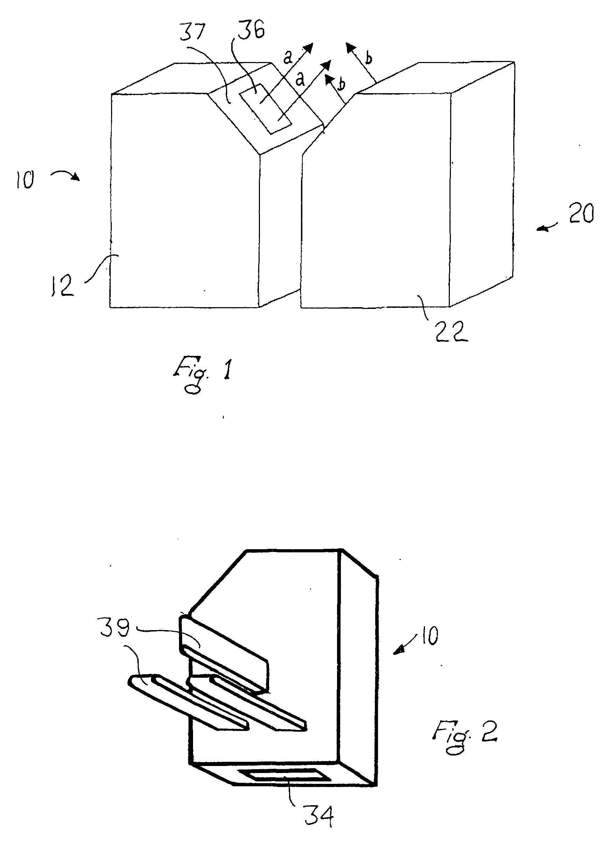

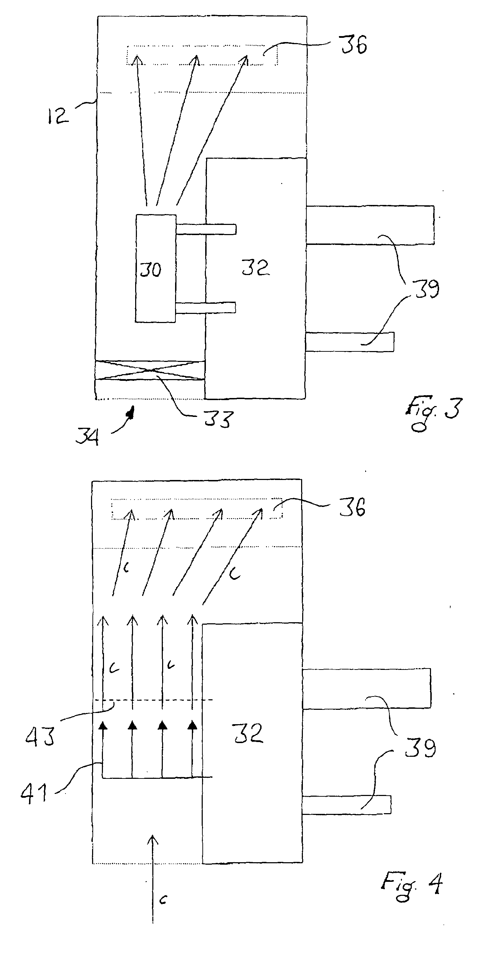

Referring to the figure 1, the system includes an ozone generating unit 10, and a vapour release unit 20, both units being connected to mains power sockets (not here shown). Referring to figure 3, the ozone generating unit comprises an ozone generator 30 mounted on a power supply 32, and a fan 33, mounted below the ozone generator. The ozone generator 30, power supply 32 and fan 33 are enclosed in a protective housing 12. A three pin plug 39 connected to the power supply 32 extends from the housing 12 which, in use, is introduced into a standard three hole mains wall socket. Figure 2 shows the air inlet 34 and the three pin plug 39 of the ozone generating unit.

The power supply 32 includes a rectifier and voltage increasing means (such as a step-up transformer or an electronic voltage multiplier). The ozone generator 30 is supplied by the power supply to produce ozone from the atmospheric oxygen by corona discharge or some similar effect.

The power supply 32 also drives the fan 33. The fan urges air into the housing through an air inlet 34 in the housing 12, over or through the ozone generator 30, and urges the ozone rich air out through the air outlet 36 in the housing.

The vapour release unit 20 comprises a heating element mounted in a housing 22, and a strip of absorbent material impregnated with terpenes which is supported near to the heating element. The heating element has a three pin plug which extends from the housing, and which in use is introduced into a standard three hole mains wall socket in the same manner as the three pin plug of the ozone generating unit.

The housings 10, 20 of the ozone generating unit and vapour release unit are similar and essentially cuboid. The air inlets (only the ozone generating unit's air outlet 36 is here visible) are located on the lower faces of the housings. Each housing also features a chamfered face (again, only the ozone generating unit's air chamfered face 37 is here visible), of an approximately 45° angle to the vertical and horizontal. On the ozone generating unit 10 this chamfered face 37 is on the left upper side of the housing (considering the pins of the three pin plug pointing forward), whilst on the vapour release unit 20 this chamfered face is on the right upper side of the housing 22.

To operate the ozone generating unit 10 and the vapour release unit 20, they are plugged side by side into a conventional double mains socket, with the ozone generating unit 10 to the left of the vapour release unit 20 (again considering the pins of the three pin plug pointing forward). Switching both sockets to the on position causes the ozone generating unit to generate ozone rich air and expel this from the outlet 36 under the influence of the fan 33. The ozone rich air is expelled approximately perpendicularly to the chamfered face 36. The heating element of the vapour release unit 20 heats the absorbent material, which starts to release the terpenes contained in the absorbent material. The hot air containing the terpenes generated by the vapour release unit 20 rises by convection through the vapour release unit, and exits at an angle of approximately 45° through the outlet on the chamfered face on the vapour release unit.

The stream of ozone-rich air (indicated by arrows a) exiting from the ozone generating unit's outlet 36 and the stream of terpenerich air (indicated by arrows b) exiting from the vapour release unit's outlet meet, causing the mixing of the two air streams. Air exiting the ozone generating unit may also aid the flow of air through the vapour release unit by somewhat reducing the air pressure immediately outside the vapour release unit's outlet.

Terpenes are chiefly derived from plants (typically, though not exclusively, coniferous plants, citrus plants and aromatic herbs). Many terpenes not only have pleasant aromas, but have disinfectant activity. The terpenes dispersed into the volume of air in the room to be treated, neutralise bacteria and viruses. Other essential oils could be used with, or instead of, the terpenes in the vapour release unit.

The ozone expelled from the ozone generating unit is also an effective disinfectant, and it too is carried into the air of the room being treated.

When terpenes and the ozone from the two air streams mix, the terpenes reduce the ozone, producing oxidised terpenes. These oxidised terpenes have a strong disinfectant activity, greater than that which would be expected solely from the combination of the ozone and terpene. Neither the unoxidised terpenes nor the oxidised terpenes are known to be harmful to people, but on the contrary are found pleasant and invigorating.

It will be seen that the ozone generating unit may be used without the vapour release unit, to produce ozone solely. Similarly, the vapour release unit may be used without the ozone generating unit to produce only essential oil vapour. The two co-operating units thus offer great flexibility. The essential oil packs must be replaced, and this makes the production of vapour relatively expensive when compared to the ozone production. The vapour release unit may be switched off overnight (when nobody is present to appreciate the scent) to conserve the terpene in the absorbent pad, whilst the ozone generating unit is left operating. The ozone generating unit may be switched off whilst the vapour release unit is left running, if, for example, someone using the room is sensitive to ozone. The units may of course be brought and used singly, in single three pin plugs, or two units may be separated and used to treat two different volumes of air. It will also be seen that the ozone generating unit could be placed (considering the pins of the three pin plug pointing forward) to the left of the vapour release unit so that the units may both operate without the mixing of the air streams.

It will be seen that although the housings described above are essentially cuboid, with a height greater than the width, the shape of the housings may be any convenient shape, and may incorporate angled and rounded surfaces.

One or both the units could include a three pin socket on the surface of the housing facing out from the wall on which it is placed. A second unit could then be plugged into the first unit. In this way, two units may be powered by a single plug. In such an arrangement, the outlet of the first unit could engage with the inlet of the second unit, so that air containing ozone and terpenes issues from the second unit's outlet.

The ozone generator unit and vapour release unit could be combined within a single housing. The inlets could be configured in various ways, for example the combined unit having a single, common inlet and outlet, or maintaining distinct inlets and outlets for the ozone generating unit and the vapour release unit. Preferably, switch means on the combined unit could be used to allow the user to switch one or both the units off.

The vapour release unit may include a fan, in the same manner as the ozone generating unit, which urges air into the inlet, over the heating element and absorbent material, and expels it from the outlet. A fan alone (that is, without a heating element), or a completely passive vapour release unit relying on evaporation and diffusion, may be sufficient to allow the volatile oils to escape the vapour release unit. Other forms of vapour release means, such as those employing electrostatics, ion flow, or a reservoir of liquid which it atomised, may be used in the vapour release unit.

Referring to figure 4, the ozone generating unit 10 may, rather than a fan, have a cathode comprising several pins 41, and an anode comprising a perforated sheet 43 as the other electrode, such that the charged ozone particles produced between the electrodes 41,43 is accelerated to the sheet electrode, a proportion passing through the holes, this effect causing an air flow sufficient to urge the ozone rich air out of the outlet as indicated by the arrows c. Naturally, other types of ozone generator could be substituted.

The inlets of the ozone generating unit and the vapour release unit may be located in other positions on the housings. The inlet of the ozone generating unit should, for maximum efficiency, be placed away from the outlet. Similarly the inlet of the vapour release unit should be placed away from the outlet. Each inlet and outlet could however be combined into a single opening, the stream of air entering the unit through one part of the opening, and the stream exiting the unit using another part of the opening. This arrangement will be more suitable for the vapour release unit, where the treated warm air and untreated cold air will be able to circulate in separate streams through a single opening provided the opening is a suitable size and shape.

The outlets of the ozone generating unit and the vapour release unit may be placed in other positions where the exiting air streams will meet and mix. The outlets could, for example, be placed in the front faces of the units (i.e. facing out from the wall on which the double socket is mounted), in upper horizontal surfaces of the housings, or in the vertical sides. The outlets could incorporate vanes or other structures to direct the air streams together and to encourage mixing.

The outlet of one of the units could be situated so that the exiting air stream was directed into the inlet of the other unit. If the ozone-rich air of the ozone generating unit is immediately taken up by the vapour release unit, or vice versa, intimate mixing of the ozone and terpenes will occur.

It will be realised that other plug and socket systems may be used to implement the present invention, for example the rounded two pin plug and socket. Also, an ozone producing unit and volatile oil producing unit could be used in such a system, wired to the power supply without a plug and socket, for example as wall mounted washroom fittings. Further, a single unit could be provided having individually controllable ozone and volatile oil producing components.

## Claims

**1.** An air treatment system including
an ozone generator contained within a first housing,
a volatile oil vaporiser contained within a second housing,
each housing including an inlet and an outlet,
the two housings being separate or separable from each other
such that in operation air enters through at least one of the inlets, and ozone and/or volatile oil exits with the air through at least one of the outlets.

**2.** An air treatment system including
an ozone generator,
a volatile oil vaporiser,
housing means for shielding and/or supporting the ozone generator and the volatile oil vaporiser, the housing means including one or more inlets and one or more outlets, and
such that in operation air enters through the inlet, and ozone and/or volatile oil exits with the air through the outlet.

**3.** An air treatment system according to any previous claim wherein the production of volatile oil and the production of ozone may be independently controlled.

**4.** An air treatment system according to any of claims 2 to 3 wherein the ozone generator is contained within a first housing, and the volatile oil vaporiser is contained within a second housing, the two housings being separate or separable from each other

**5.** An air treatment system according to any of claims 1, 3 or 4 wherein each housing including plug means.

**6.** An air treatment system according to any of claims 1 or 3 to 5 wherein air containing ozone exits from the outlet of the first housing, and air containing volatile oil exits from a outlet of the second housing, the outlets directing their air flows together so as to mix in the surrounding air.

**7.** An air treatment system according to any of claims 1 or 3 to 5 wherein air containing ozone exits from a outlet of the first housing and is directed so that a substantial proportion is taken up by the inlet of the second housing, so that this air exits through the second outlet.

**8.** An ozone generator adapted to co-operate with a volatile oil vaporiser as defined in any previous claim.

**9.** A volatile oil vaporiser adapted to co-operate with an ozone generator as defined in any of the claims 1 to 8.

**9.** An air treatment system according to any previous claim wherein the volatile oils include terpenes.

**10.** An ozone generator adapted to co-operate with an volatile oil vaporiser as defined in any previous claim.

**11.** A volatile oil vaporiser adapted to co-operate with an ozone generator as defined in any of the claims 1 to 11.

**12.** A method of treating air in a room or the like by generating a first air stream containing ozone, generating a second air stream containing volatile oil, and directing the air streams so as to converge and mix.
